# EUROPÄISCHE PATENTSCHRIFT

(11) **EP 1 985 317 B1**
(45) Veröffentlichungstag und Bekanntmachung des Hinweises auf die Patenterteilung: **05.06.2013**
(21) Anmeldenummer: 08007307.5
(22) Anmeldetag: 14.04.2008
(51) Int. Cl.: A61L 24/00

(54) **Spacer-Polymethylmethacrylat-Knochenzement**
Spacer polymethyl methacrylate bone cement
Ciment osseux d'espaceur en polyméthylméthacrylate

(30) Priorität: 24.04.2007 DE 102007019593; 21.06.2007 DE 102007029098
(43) Veröffentlichungstag der Anmeldung: 29.10.2008
(73) Patentinhaber: Heraeus Kulzer GmbH, 63450 Hanau (DE)
(72) Erfinder: Vogt, Dr. Sebastian, 99092 Erfurt (DE); Büchner, Dr. Hubert, 64354 Reinheim (DE); Kühn, Dr. Klaus-Dieter, 35041 Marburg-Einhausen (DE); Gopp, Dr. Udo, 61273 Wehrheim (DE); Thomsen, Professor Dr. Marc, 69117 Heidelberg (DE)
(74) Vertreter: Kühn, Hans-Christian

(56) Entgegenhaltungen:
- EP-A2- 1 757 272
- DE-A1- 2 327 126
- US-A1- 2005 106 528
- TOLGA TUZUNER ET AL: "Elution characteristics and mechanical properties of calcium sulfate-loaded bone cement containing teicoplanin", JOURNAL OF ORTHOPAEDIC SCIENCE ; OFFICIAL JOURNAL OF THE JAPANESE ORTHOPAEDIC ASSOCIATION, SPRINGER-VERLAG, TO, Bd. 12, Nr. 2, 30. März 2007 (2007-03-30), Seiten 170-177, XP019519894, ISSN: 1436-2023, DOI: DOI:10.1007/S00776-006-1107-9

## Beschreibung

Gegenstand der Erfindung ist ein Spacer-Polymethylmethacrylat-Knochenzement, der für die Herstellung von temporären Platzhaltern für die zweizeitige Revision von Gelenkendoprothesen geeignet ist.

Gelenkendoprothesen haben gegenwärtig eine Standzeit von mehreren Jahren, zum Beispiel bei zementierten Hüftgelenkprothesen im Durchschnitt größer 10-15 Jahre. Es gibt jedoch unerwünschte Lockerungen der Gelenkendoprothesen, die vor dem Erreichen der üblichen Standzeiten auftreten. Es wird dabei die septische und die aseptische Lockerung unterschieden. Bei der aseptischen Lockerung lassen sich bisher keine mikrobiellen Keime nachweisen. Die Ursachen der aseptischen Lockerungen können vielfältig sein. Häufig sind aseptische Lockerungen auf Abrieb an den Gleitflächen der Gelenkendoprothesen zurückzuführen. Bei der septischen Lockerung wird der Lockerungsprozess durch mikrobielle Keime hervorgerufen. Man unterscheidet dabei in Abhängigkeit vom zeitlichen Auftreten frühe und späte Infektionen. Die septische Lockerung ist eine sehr ernste Erkrankung für den Patienten, die zusätzlich mit sehr hohen Kosten verbunden ist. Sowohl bei der aseptischen als auch der septischen Lockerung wird üblicherweise eine Revision vorgenommen. Man unterscheidet dabei die einzeitige und die zweizeitige Revision.

Bei der zweizeitigen Revision wird im Allgemeinen ein Platzhalter, ein so genannter Spacer, eingesetzt. Dieser Spacer füllt für einige Wochen den Raum der zuvor revidierten Endoprothese aus, bis die vorliegende Infektion abgeklungen ist. Diese Platzhalterfunktion ist sehr wichtig, um ein Schrumpfung der Muskulatur in dieser Zeit wirksam zu verhindern und um eine Stabilisierung der Resektionssituation zu erreichen. Weiterhin wird durch artikulierende Spacer die Beweglichkeit der betroffenen Gliedmaßen erhalten. Es ist dadurch möglich, die Patienten frühzeitig zu mobilisieren.

Spacer werden üblicherweise vom Chirurgen aus konventionellen PMMA-Knochenzementen unter Nutzung geeigneter Formen selbst hergestellt. Dabei werden dem PMMA-Knochenzement-Pulver vor der Spacer-Herstellung entsprechend den beo Punktionen vorgefundenen mikrobiellen Keimen und nach erfolgtem Antibiogramm ein oder mehrere Antibiotika zugemischt. Die Antibiotika-Auswahl wird speziell auf die vorliegenden mikrobiellen Keime abgestimmt. Dieses Vorgehen ist insbesondere beim Auftreten von mehrfach resistenten Keimen oder bei Mischinfektionen mit unterschiedlichen Keimen sehr vorteilhaft.

Die Entwicklung von Spacern geht ursprünglich auf Hovelius und Josefsson zurück (Hovelius L, Josefsson G (1979), An alternative method for exchange operation of infected arthroplasty. Acta Orthop Scand 50: 93-96). Weitere frühe Arbeiten zu Spacern stammen von Younger (Younger AS, Duncan CP, Masri BA, McGraw RW (1997), The outcome of two-stage arthroplasty using a custom-made interval spacer to treat the infected hip. J Arthroplasty 12: 615-623), Jones (Jones WA. Wroblewski BM (1989) Salvage of failed total knee arthroplasty: the 'beefburger' procedure. J Bone Joint Surg Br. 71: 856-857.) und Cohen (Cohen JC, Hozack WJ, Cuckler JM, Booth RE Jr (1988), Two-stage reimplantation of septic total knee arthroplasty. Report of three cases using an antibiotic-PMMA spacer block. J Arthroplasty 3: 369-377).
Von McPherson stammt die Konzeption, dass ausschließlich Spacer aus Knochenzement hergestellt werden und eine Reimplantion von originalen Prothesenteilen unterbleibt (McPherson EJ, Lewonowski K, Dorr LD (1995), Techniques in arthroplasty. Use of an articulated PMMA spacer in the infected total knee arthroplasty. J. Arthroplasty 10: 87-89).

Problematisch ist bei den bisher eingesetzten Spacern, dass diese durch die im zu Grunde liegenden PMMA-Knochenzement enthaltenen sehr harten Röntgenopaker-Partikel, wie Zirkoniumdioxid und Bariumsulfat, eine gewisse Abrasion zeigen. Abrasionserscheinungen sind insbesondere bei artikulierenden Spacern an den Gleitflächen sehr kritisch zu sehen. Es wird gegenwärtig diskutiert, ob eventuell der beim Einsatz von Spacern entstehende Abrieb eine aseptische Lockerung der Revisionsendoprothesen bei der zweizeitigen Revision verursachen kann.

Ein weiteres Problem der bisher eingesetzten Spacer besteht darin, dass die im PMMA-Knochenzement eingeschlossenen Antibiotika-Partikel nur oberflächlich durch Einwirkung von Körperflüssigkeit herausgelöst werden. Um eine hohe initiale Freisetzung zu bekommen, werden deshalb üblicherweise sehr große Mengen Antibiotika zugesetzt, die bei normalen PMMA-Knochenzementen zur dauerhaften Fixierung von Totalgelenkendoprothesen nicht üblich sind. Angestrebt wird eine Freisetzung größerer Antibiotika-Mengen über einen Zeitraum von mehreren Tagen bis einigen Wochen.

Es wurde im DE 2905878 offenbart, dass durch einen Zusatz von Kochsalz oder anderen löslichen Alkalihalogeniden die Antibiotika-Freisetzung aus PMMA-Knochenzementen erhöht werden kann. Alternativ dazu wurde in US 4233287 vorgeschlagen, in Wasser lösliche Aminosäuren in PMMA-Zemente zur Verbesserung der Wirkstofffreisetzung zu inkorporieren. Der wesentliche Nachteil dieser beiden Methoden besteht darin, dass bei der Verwendung von größeren Mengen an in Wasser löslichen Alkalihalogeniden bzw. Aminosäuren in PMMA-Knochenzementen bei Einwirkung von Wundsekret bzw. Blut auf den ausgehärteten Knochenzement durch Herauslösung dieser Additive lokal hypertonische Lösungen entstehen können, die unphysiologisch sind.

Sencan et al. untersuchte die bakterielle Adhärenz auf PMMA-Knochenzement der Teicoplanin und Calciumsulfat enthielt (I. Sencan, I. Sahn, T. Tuzuner, D. Özdemir, M. Yildirim, H. Leblebicioglu: In vitro bacterial adherence to teicoplanin and calcium sulfat-soaked bone cement. J. Chemother. 17 (2005) 174-178.). Er fand eine Freisetzung größerer Mengen Teicoplanin im wässrigen Medium in den ersten drei Tagen und danach eine Freisetzung geringerer Mengen Teicoplanin bis zu 33 Tagen.

Tuzuner et al. (Elution characteristics and mechanical properties of calcium sulfate-loaded bone cement containing teicoplanin. J. Orthop. Sci. 12 (2007) 170-177) beschreiben auch einen PMMA- Knochenzement umfassend Calciumsulfat-Dihydrat und Teicoplanin, der in der chirurgischen Revision von Gelenkendoprothesen (z.B. Hüftgelenkprothesen) Anwendung findet. Durch den Zusatz von Calciumsulfat- Dihydrat wird die Antibiotika-Freisetzung aus PMMA-Knochenzement erhöht.

Der Erfindung liegt die Aufgabe zu Grunde, einen Polymethylmethacrylat-Knochenzement für die Herstellung von temporären Platzhaltern zu entwickeln, der einerseits keine größere Mengen an harten Abriebpartikeln freisetzen kann und der andererseits eine hohe Antibiotikum/Antibiotika-Freisetzung bei Einwirkung von wässrigen Medien, wie Wundsekret oder Blut, zeigt. Der zu entwickelnde Polymethylmethacrylat-Knochenzement soll so beschaffen sein, dass auch Antibiotika in tieferen Bereichen des Knochenzementes durch Einwirkung von wässrigen Körperflüssigkeiten herausgelöst werden können.

Die Aufgabe wurde erfindungsgemäß durch einen Polymethylmethacrylat-Knochenzement gelöst, der dadurch charakterisiert ist, dass ein hydrolytisch degradierbarer Röntgenopaker mit einer Härte nach Mohs von gleich kleiner 3 und einer Löslichkeit in Wasser bei Raumtemperatur von kleiner 4 g pro Liter enthalten ist, wobei der hydrolytish degradierbare Röntgenopaker aus der Gruppe ausgewählt ist, die aus Calciumcarbonat, Magnesiumcarbonat und Gemischen davon besteht.

Der hydrolytisch degradierbare Röntgenopaker ist bevorzugt mikroporös und enthält gegebenenfalls einen pharmazeutischen Hilfsstoff enthält.

Zusätzlich zu dem hydrolytisch degradierbaren Röntgenopaker kann Zirkoniumdioxid, Bariumsulfat oder Tantal enthalten sein.

Die Gesamtmenge an Röntgenopaker beträgt bevorzugt von 5 - 25 Gew%.
Der hydrolytisch degradierbarer Röntgenopaker mit einer Härte nach Mohs von gleich kleiner 3 und einer Löslichkeit in Wasser bei Raumtemperatur von kleiner 4 g pro Liter bevorzugt zu 3 bis 12 % vorhanden ist.

Als hydrolytisch degradierbarer Röntgenopaker sind Calciumcarbonat, Magnesiumcarbonat, und Gemischen davon. Calciumcarbonat (Calcit) hat eine Härte nach Mohs von 3 und ist somit ein sehr welcher Röntgenopaker. Besonders vorteilhaft ist, dass Calciumcarbonat üblicherweise kein Kristallwasser enthält, dass eventuell bei der bei PMMA-Knochenzementen üblichen Sterilisation mit Ethylenoxid eine Nebenreaktion unter der Bildung von Ethylenglykol eingehen kann. Calciumcarbonat löst sich in Gegenwart von Kohlendioxid gesättigten wässrigen Lösungen, wie sie im humanen Organismus z. B. im Blut vorliegen, durch Einwirkung von Hydrogencarbonat.

Das Calciumcarbonat kann geringe Mengen von physiologisch unbedenklichen Strontiumsalzen und Magnesiumsalzen, wie zum Beispiel Strontiumsulfat, Strontiumcarbonat und Magnesiumcarbonat, enthalten.

Die Erfindung betrifft auch die Verwendung des beschriebenen Polymethylmethacrylat-Knochenzements als temporären Platzhalter.

Der beschriebene Polymethylmethacrylat-Knochenzement kann auch zur dauerhaften Fixierung von Gelenkendoprothesen verwendet werden. Der Knochenzement ist zur Implantation von üblichen Hüft-, Knie- und Schultergelenken prinzipiell geeignet. Daneben ist es auch möglich, aus dem erfindungsgemäßen Knochenzement flächige Implantate herzustellen, die Verwendung bei der Rekonstruktion von Knochendefekten im Hirn- und Gesichtsschädelbereich finden können. Ebenfalls ist es grundsätzlich möglich, den Knochenzement zur Vertebroplastie und zur Kyphoplastie einzusetzen.

Die Erfindung wird durch die nachstehenden Beispiele näher erläutert, ohne jedoch die Erfindung zu beschränken.

### Beispiele

Es werden zuerst 17 Zementpulver durch Vermahlung hergestellt. Die Zusammensetzung ist in der nachfolgenden Tabelle angegeben. Die Beispiele 1-9 dienen dabei als Referenz.

| Beispiel-Nr. | Zusammensetzung des Zementpulvers | | | | |
|---|---|---|---|---|---|
| | Dibenzoyl-peroxid | Polymethylmethacrylat-comethylacrylat | ZrO₂ | CeSO₄x 2H₂O | Gentamicinsulfat (AK600) |
| 1 | 0,4 g | 33,7 g | 5,9 g | - | 1,66 g (entspricht 1,0 g Gentamicinbase) |
| 2 | 0,4 g | 33,7 g | 5,9 g | - | 3,33 g (entspricht 2,0 g Gentamicinbase) |
| 3 | 0,4 g | 33,7 g | 5,9 g | - | 6,66 g (entspricht 4,0 g Gentamicinbase) |
| 4 | 0,4 g | 33,7 g | 4,0 g | 1,9 g | 1,66 g (entspricht 1,0 g Gentamicinbase) |
| 5 | 0,4 g | 33,7 g | 4,0 g | 1,9 g | 3,33 g (entspricht 2,0 g Gentamicinbase) |
| 6 | 0,4 g | 33,7 g | 4,0 g | 1,9 g | 6,66 g (entspricht 4,0 g Gentamicinbase) |
| 7 | 0,4 g | 33,7 g | 2,0 g | 3,9 g | 1,66 g (entspricht 1,0 g Gentamicinbase) |
| 8 | 0,4 g | 33,7 g | 2,0 g | 3,9 g | 3,33 g (entspricht 2,0 g Gentamicinbase) |
| 9 | 0,4 g | 33,7 g | 2,0 g | 3,9 g | 6,66 g (entspricht 4,0 g Gentamicinbase) |

| Dibenzoyl-peroxid | Polymethylmethacrylat-comethylacrylat | ZrO₂ | Opaker | Gentamicinsulfat (AK600) | |
|---|---|---|---|---|---|
| 10 | 0,4 g | 33,6 g | 4,0 g | 2,0 g CaCO₃ | 3,33 g (entspricht 2,0 g Gentamicinbase) |
| 11 | 0,4 g | 33,6 g | 4,0 g | 2,0 g MgCO₃ | 3,33 g (entspricht 2,0 g Gentamicinbase) |
| 12 | 0,4 g | 33,6 g | 4,0 g | 1,0 g CaSO₄x 2H₂O + 1,0 g CaCO₃ | 3,33 g (entspricht 2,0 g Gentamicinbase) |
| 13 | 0,4 g | 33,6 g | 4,0 g | 1,0 g CaSO₄x 2H₂O + 1,0 g MgCO₃ | 3,33 g (entspricht 2,0 g Gentamicinbase) |
| 14 | 0,4 g | 33,6 g | 2,0 g | 4,0 g CaCO₃ | 3,33 g (entspricht 2,0 g Gentamicinbase) |
| 15 | 0,4 g | 33,7 g | 2,0 g | 4,0 g MgCO₃ | 3,33 g (entspricht 2,0 g Gentamicinbase) |
| 16 | 0,4 g | 33,7 g | 2,0 g | 2,0 g CaSO₄x 2H₂O + 2,0 g CaCO₃ | 3,33 g (entspricht 2,0 g Gentamicinbase) |
| 17 | 0,4 g | 33,7 g | 2,0 g | 2,0 g CaSO₄x 2H₂O + 2,0 g MgCO₃ | 3,33 g (entspricht 2,0 g Gentamicinbase) |

Danach wird jeweils 40 g Zementpulver mit 20 ml Methylmethacrylat vermischt, in dem 1,0 Ma% Dimethyl-p-toluidin gelöst sind. Es bildet sich ein Teig, der in Hohlformen gestrichen wird und dort nach wenigen Minuten aushärtet. Die entstehenden zylinderförmigen Probekörper haben eine Höhe von 1 cm und einen Durchmesser von 2,5 cm. Es werden jeweils 5 Probekörper pro Zementvariante hergestellt. Die Probekörper werden separat in jeweils 20 ml destilliertem Wasser bei 37 °C eingelagert. Täglich wird das Freisetzungsmedium vollständig entnommen und darin die freigesetzte Gentamicinmenge bestimmt. Die Probekörper werden dann wieder in jeweils 20 ml frischem destillierten Wasser bei 37 °C gelagert. Die Bestimmung des Gentamicingehaltes im Eluat erfolgt mit einem TDX-Analyser der Firma Abott. Die jeweils freigesetzte Masse an Gentamicinbase wird pro Probekörper in der nachfolgenden Tabelle in Abhängigkeit von dertagerungszeit der Probekörper im Freisetzungsmedium angegeben.

| Proben-Nr. | Gentamicinfreisetzung pro Formkörper [µg/Formkörper] | | |
|---|---|---|---|
| | 1 d | 3 d | 5d |
| 1 | 1.806 | 74 | 45 |
| 2 | 4.568 | 191 | 141 |
| 3 | 14.386 | 1.507 | 888 |
| 4 | 1.979 | 99 | 122 |
| 5 | 4.672 | 370 | 293 |
| 6 | 18.887 | 2.545 | 1.529 |
| 7 | 2.476 | 134 | 75 |
| 8 | 6.073 | 497 | 286 |
| 9 | 22.602 | 2.565 | 1.659 |
| 10 | 4818 | 367 | 325 |
| 11 | 5169 | 420 | 460 |
| 12 | 5294 | 391 | 353 |
| 13 | 6665 | 515 | 598 |
| 14 | 6344 | 836 | 593 |
| 15 | 6877 | 693 | 478 |
| 16 | 5202 | 415 | 442 |
| 17 | 6166 | 391 | 323 |

Zusätzlich werden mit den Zementen der Beispiele 1-17 Platten hergestellt und daraus Streifen geschnitten. Die 4-Punkt-Biegefestigkeit und das E-Modul werden anschließend an diesen Streifen bestimmt. Die Ergebnisse der sind in nachfolgender Tabelle dargestellt. Übliche zur Fixierung von Gelenkendoprothesen eingesetzte PMMA-Knochenzemente sollten mindestens eine Biegefestigkeit beim 4-Punkt-Biegeversuch von ≥ 50 MPA und ein E-Modul ≥ 1800 MPA haben. Die Ergebnisse zeigen, dass die Mindestanforderungen an die Biegefestigkeit und an das E-Modul von allen Zementen bis auf den Zement der Proben-Nr. 9 erfüllt wurden. Eine Ausnahme bildet der Zement des Beispiel 9, bei dem die Biegefestigkeit um ca. 5 MPa unterschritten wird. Auch dieser Befund ist für einen Spacer-PMMA-Knochenzement durchaus akzeptabel, weil der Spacer-PMMA-Knochenzement nur temporär implantiert ist und keine dauerhafte Festigkeit aufweisen muss.

| Proben-Nr. | 4-Punkt-Biegung | |
|---|---|---|
| | Biegefestigkeit [MPa] | E-Modul [MPa] |
| 1 | 60,9 | 2516 |
| 2 | 60,8 | 2651 |
| 3 | 55,4 | 2657 |
| 4 | 61,3 | 2.722 |
| 5 | 53,9 | 2.654 |
| 6 | 51,2 | 2.826 |
| 7 | 52,1 | 2.768 |
| 8 | 54,9 | 2.728 |
| 9 | 45,3 | 2.671 |
| 10 | 61,5 | 2686 |
| 11 | 58,9 | 2859 |
| 12 | 61,2 | 2867 |
| 13 | 56,7 | 2773 |
| 14 | 60,7 | 2859 |
| 15 | 55,6 | 2917 |
| 16 | 59,7 | 2923 |
| 17 | 53,8 | 2863 |

Es wurden weiterhin drei Zemente ohne Antibiotikum hergestellt und deren Biegefestigkeit und Biegemodul bestimmt. Das Beispiel 20 dient dabei als Referenz.

| Beispiel-Nr. | Dibenzoyl-peroxid | Polymethylmethacrylat-comethylacrylat | Opaker |
|---|---|---|---|
| 18 | 0,4 g | 33,6 g | 6,0 g CaCO₃ |
| 19 | 0,4 g | 33,6 g | 6,0 g MgCO₃ |
| 20 | 0,4 g | 33,6 g | 6,0 g CaSO₄x 2H₂O |

| Proben-Nr. | 4-Punkt-Biegung | | |
|---|---|---|---|
| | Biegefestigkeit [MPa] | E-Modul [MPa] | |
| 18 | 58,5 | 2820 | |
| 19 | 58,9 | 2702 | |
| 20 | 60,0 | 2619 | |

Es wurde nachfolgend auch Spacer-PMMA-Knochenzemente mit Bariumsulfat und mit Tantal als zusätzliche Röntgenopaker hergestellt. Es wurden dabei gepulvertes Bariumsulfat und Tantal-Staub eingesetzt. Die Zemente der Beispiel 21 und 24 ließen sich problemlos vermischen und zeigten einen vergleichbare Wirkstofffreisetzung wie die Probekörper des Beispiels 7. Die Beispiele 21 und 22 dienen dabei als Referenz.

| Beispiel-Nr. | Zusammensetzung des Zementpulvers | | | | |
|---|---|---|---|---|---|
| | Dibenzoyl-peroxid | Polymethylmethacrylat-comethylacrylat | Opaker | Degradierbarer Opaker | Gentamicinsulfat (AK600) |
| 21 | 0,4 g | 33,7 g | 2,0 g Bariumsulfat | 3,9 g CaSO₄x 2H₂O | 1,66 g (entspricht 1,0 g Gentamicinbase) |
| 22 | 0,4 g | 33,7 g | 2,0 g Tantal-Pulver | 3,9 g CaSO₄x 2H₂O | 1,66 g (entspricht 1,0 g Gentamicinbase) |
| 23 | 0,4 g | 33,7 g | 2,0 g Bariumsulfat | 3,9 g Ca-CO₃ | 1,66 g (entspricht 1,0 g Gentamicinbase) |
| 24 | 0,4 g | 33,7 g | 2,0 g Tantal-Pulver | 3,9 g MgCO₃ | 1,66 g (entspricht 1,0 g Gentamicinbase) |

## Patentansprüche

1. Polymethylmethacrylat-Knochenzement, **dadurch gekennzeichnet, dass** ein hydrolytisch degradierbarer Röntgenopaker mit einer Härte nach Mohs von 3 oder weniger und einer Löslichkeit in Wasser bei Raumtemperatur von kleiner 4 g pro Liter enthalten ist, wobei der hydrolytisch degradierbare Röntgenopaker aus der Gruppe ausgewählt ist, die aus Calciumcarbonat, Magnesiumcarbonat und Gemischen davon besteht.

2. Polymethylmethacrylat-Knochenzement nach Anspruch 1, **dadurch gekennzeichnet, dass** der hydrolytisch degradierbare Röntgenopaker mikroporös ist und gegebenenfalls einen pharmazeutischen Hilfsstoff enthält.

3. Polymethylmethacrylat-Knochenzement nach Anspruch 1 oder 2, **dadurch gekennzeichnet, dass** zusätzlich zu dem hydrolytisch degradierbaren Röntgenopaker Zirkoniumdioxid, Bariumsulfat oder Tantal enthalten ist.

4. Polymethylmethacrylat-Knochenzement nach mindestens einem der vorstehenden Ansprüche, **dadurch gekennzeichnet, dass** Röntgenopaker insgesamt zu 5 - 20 Gew% vorhanden ist.

5. Verfahren zur Herstellung eines temporären Platzhalters, **dadurch gekennzeichnet, dass** ein Polymethylmethacrylat-Knochenzement nach einem der vorstehenden Ansprüche verwendet wird.

## Claims

1. Polymethylmethacrylate bone cement, **characterised in that** it contains a radiopaquer that can be degraded hydrolytically and has a Mohs hardness of 3 or less and a solubility in water at room temperature of less than 4 g per litre, whereby the radiopaquer that can be degraded hydrolytically is selected from the group consisting of calcium carbonate, magnesium carbonate, and mixtures thereof.

2. Polymethylmethacrylate bone cement according to claim 1, **characterised in that** the radiopaquer that can be degraded hydrolytically is microporous and, if applicable, contains a pharmaceutical expedient.

3. Polymethylmethacrylate bone cement according to claim 1 or 2, **characterised in that** it contains zirconium dioxide, barium sulfate or tantalum in addition to the radiopaquer that can be degraded hydrolytically.

4. Polymethylmethacrylate bone cement according to at least one of the preceding claims, **characterised in that** a total of at least 5 - 20 % by weight of the radiopaquer is present.

5. Method for producing a temporary spacer, **characterised in that** a polymethylmethacrylate bone cement according to any one of the preceding claims is used.

## Revendications

1. Ciment osseux en polyméthylméthacrylate, **caractérisé en ce qu'**un radio-opacifiant pouvant être dégradé par voie hydrolytique avec une dureté de Mohs de 3 ou moins et une solubilité dans l'eau à température ambiante inférieure à 4 g par litre est contenu, dans lequel le radio-opacifiant pouvant être dégradé par voie hydrolytique est sélectionné parmi le groupe qui se compose du carbonate de calcium, du carbonate de magnésium et de mélanges de ceux-ci.

2. Ciment osseux en polyméthylméthacrylate selon la revendication 1, **caractérisé en ce que** le radio-opacifiant pouvant être dégradé par voie hydrolytique est microporeux et contient éventuellement un adjuvant pharmaceutique.

3. Ciment osseux en polyméthylméthacrylate selon la revendication 1 ou 2, **caractérisé en ce que**, outre le radio-opacifiant pouvant être dégradé par voie hydrolytique, du dioxyde de zirconium, du sulfate de baryum ou du tantale est contenu.

4. Ciment osseux en polyméthylméthacrylate selon au moins une des revendications précédentes, **caractérisé en ce que** le radio-opacifiant est présent au total à 5 à 20 % en poids.

5. Procédé de fabrication d'un mainteneur d'espace temporaire, **caractérisé en ce qu'**un ciment osseux en polyméthylméthacrylate selon l'une quelconque des revendications précédentes est utilisé.
